(19) Europäisches Patentamt
European Patent Office
Office européen des brevets

(11) Veröffentlichungsnummer: **0 144 012**
**B1**

(12) # EUROPÄISCHE PATENTSCHRIFT

(45) Veröffentlichungstag der Patentschrift: **16.03.88**

(51) Int. Cl.[4]: **C 07 D 401/12**, C 07 D 401/06, A 61 K 31/40

(21) Anmeldenummer: **84113621.1**

(22) Anmeldetag: **12.11.84**

(54) **Indolderivate.**

(30) Priorität: **25.11.83 DE 3342632**

(43) Veröffentlichungstag der Anmeldung: **12.06.85 Patentblatt 85/24**

(45) Bekanntmachung des Hinweises auf die Patenterteilung: **16.03.88 Patentblatt 88/11**

(84) Benannte Vertragsstaaten: **AT BE CH DE FR GB IT LI NL SE**

(56) Entgegenhaltungen:
**EP-A-0 007 399**
**EP-A-0 105 397**
**EP-A-0 114 603**
**CH-A-462 813**
**FR-A-1 545 887**
**GB-A-1 012 618**
**US-A-3 639 414**
**US-A-4 464 379**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Böttcher, Henning, Dr., Soderstrasse 95, D-6100 Darmstadt (DE)**
Erfinder: **Hausberg, Hans- Heinrich, Dr., Odenwaldstrasse 30, D-6105 Ober- Ramstadt (DE)**
Erfinder: **Seyfried, Christoph, Dr., Mathildenstrasse 6, D-6104 Seeheim- Jugenheim (DE)**
Erfinder: **Minck, Klaus- Otto, Dr., Büchestrasse 8, D-6105 Ober- Ramstadt (DE)**

**EP 0 144 012 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Indolderivate der allgemeinen Formel I

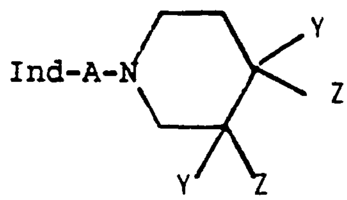

I

worin

Ind einen durch eine Hydroxymethyl- oder COW-Gruppe substituierten Indol-3-yl-rest, der zusätzlich ein- oder zweifach durch Alkyl, O-Alkyl, OH, F, Cl oder Br substituiert sein kann,
W H, OH, OAlkyl, $NH_2$, NHAlkyl oder $N(Alkyl)_2$,
A $-(CH_2)_n-$, $-CH_2-S-CH_2CH_2-$, $-CH_2-SO-CH_2CH_2-$ oder $-CH_2-SO_2-CH_2CH_2-$,
n 2, 3, 4 oder 5,
die beiden Reste Y jeweils H oder zusammen eine C-C-Bindung,
der eine Rest Z Ar,
der andere Rest Z H und
Ar eine unsubstituierte oder eine ein- oder zweifach durch O-Alkyl und/oder OH oder durch eine Methylendioxygruppe substituierte Phenylgruppe oder eine 2- oder 3-Thienylgruppe

bedeuten,
worin die Alkylgruppen jeweils 1 - 4 C-Atome besitzen,
worin jedoch n nur dann 2 oder 3 bedeutet, wenn gleichzeitig die Hydroxymethyl- oder COW-Gruppe in der 4-, 5-, 6- oder 7-Stellung des Indol-3-yl-rests steht, sowie deren physiologisch unbedenkliche Säureadditionssalze.

Ähnliche Verbindungen sind aus der US-PS-3 639 414 und der AU-PS-36 833/83 bekannt, ferner aus der EP-A-105 397 und der EP-A-114 603.

Der Erfindung lag die Aufgabe zugrunde, neue Verbindungen aufzufinden, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem dopaminstimulierende präsynaptische (neuroleptische) oder postsynaptische (Anti-Parkinson) Wirkungen. Im einzelnen induzieren die Verbindungen der Formel I contralaterales Drehverhalten in Hemiparkinson-Ratten (feststellbar nach der Methode von Ungerstedt et al., Brain Res. 24 (1970), 485-493) und hemmen die Bindung von tritiierten Dopaminagonisten und -antagonisten an striäre Rezeptoren (feststellbar nach der Methode von Schwarcz et al., J. Neurochemistry 34 (1980), 772-778 und Creese et al., European J. Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21 (1973), 178-182, und von Ilhan et al., European J. Pharmacol. 33 (1975), 61-64). weiterhin treten analgetische und blutdrucksenkende Wirkungen auf; so wird der bei Katheter-tragenden wachen, spontan hypertonen Ratten (Stamm SHR/NIH-MO/CHB-EMD; Methode vgl. Weeks und Jones, Proc. Soc. Exptl. Biol. Med. 104 (1960), 646-648) direkt gemessene Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Indolderivate der Formel I sowie ihre physiologisch unbedenklichen Säureadditionssalze.

In den Resten Ind, W und Ar bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy.

Der Rest Ind bedeutet insbesondere einen einfach substituierten Indol-3-ylrest. Vorzugsweise ist er in der 5- oder 6-Stellung oder auch in der 4- oder 7-Stellung substituiert. Weiterhin ist eine Substitution in 1- oder 2-Stellung möglich. Bevorzugte disubstituierte Indol-3-yl-reste sind in 5,6-Stellung substituiert; Disubstitution ist auch in 1,2-, 1,4-, 1,5-, 1,6-, 1,7-, 2,4-, 2,5-, 2,6-, 2,7-, 4,5-, 4,6-, 4,7-, 5,7- oder 6,7-Stellung möglich. In allen diesen Fällen können die Substituenten gleich oder verschieden sein.

Im einzelnen sind die bevorzugten Substituenten im Benzolring des Restes Ind Hydroxymethyl, Formyl, Carboxy, Methoxy- oder Ethoxycarbonyl, Carbamoyl, N-Methyl-, N-Ethyl-, N,N-Dimethyl- und N,N-Diethylcarbamoyl, in zweiter Linie Propoxy-, Isopropoxy-, Butoxy-, Isobutoxy-, sek.-Butoxy- und tert.-Butoxycarbonyl, N-Propyl-, N-Isopropyl-, N-Butyl-, N-Isobutyl-, N-sek.-Butyl-, N-tert.-Butyl-, N-Methyl-N-ethyl-, N,N-Dipropyl-, N-Methyl-N-propyl-, N-Ethyl-N-propyl- und N,N-Dibutylcarbamoyl, ferner zusätzlich Methyl,

Ethyl, Methoxy, Ethoxy, OH, F, Cl und/oder Br. Einige bevorzugte Bedeutungen des Restes Ind sind dementsprechend 2-, 4-, 5-, 6- oder 7-Formyl-indol-3-yl, 2-, 4-, 5-, 6- oder 7-Carboxy-indol-3-yl, 2-, 4-, 5-, 6- oder 7-Methoxycarbonyl-indol-3-yl, 2-, 4-, 5-, 6- oder 7-Ethoxycarbonyl-indol-3-yl, 2-, 4-, 5-, 6- oder 7-Carbamoylindol-3-yl, 2-, 4-, 5-, 6- oder 7-N-Methylcarbamoylindol-3-yl, 2-, 4-, 5-, 6- oder 7-N-Ethylcarbamoylindol-3-yl, 2-, 4-, 5-, 6- oder 7-N,N-Dimethylcarbamoyl-indol-3-yl, 2-, 4-, 5-, 6- oder 7-N,N-Diethylcarbamoylindol-3-yl,

1-Methyl-4-, -5-, -6- oder -7-hydroxymethylindol-3-yl, 1-Methyl-4-, -5-, -6- oder -7-formylindol-3-yl, 1-Methyl-4-, -5-, -6- oder -7-carboxyindol-3-yl, 1-Methyl-4-, -5-, -6- oder -7-carbamoylindol-3-yl, 2-Methyl-4-, -5-, -6- oder -7-hydroxymethylindol-3-yl, 2-Methyl-4-, -5-, -6- oder -7-formylindol-3-yl, 2-Methyl-4-, -5-, -6- oder -7-carboxyindol-3-yl, 2-Methyl-4-, -5-, -6- oder -7-carbamoylindol-3-yl, 5-Methoxy-4-, -6- oder -7-methoxycarbonylindol-3-yl, 5-Methoxy-4-, -6- oder -7-ethoxycarbonylindol-3-yl, 5-Methoxy-4-, -6- oder -7-carboxyindol-3-yl, 5-Methoxy-4-, -6- oder -7-carbamoylindol-3-yl, 5-Fluor-4-, -6- oder -7-carboxyindol-3-yl, 5-Chlor-4-, -6- oder -7-carboxyindol-3-yl, 7-Chlor-4-, -5- oder -6-carboxyindol-3-yl, 5-Brom-4-, -6- oder -7-carboxyindol-3-yl, 5-Hydroxy-4-, -6- oder -7-methoxycarbonylindol-3-yl, 5-Hydroxy-4-, -6- oder -7-ethoxycarbonylindol-3-yl, 5-Hydroxy-4-, -6- oder -7-carboxyindol-3-yl, 5-Hydroxy-4-, -6- oder -7-carbamoylindol-3-yl. 5-Hydroxy-2-, -4-, -6- oder -7-hydroxymethylindol-3-yl, 6-Hydroxy-4-, -5- oder -7-carboxyindol-3-yl, 6-Hydroxy-2-, -4-, -5- oder -7-hydroxymethylindol-3-yl.

Der Parameter n ist vorzugsweise 4, der Rest A ist vorzugsweise $-(CH_2)_4-$ oder $-CH_2-S-CH_2CH_2-$, weiterhin vorzugsweise $-(CH_2)_2-$, $-(CH_2)_3-$ oder $-(CH_2)_5-$.

Der Rest Ar ist bevorzugt unsubstituiertes Phenyl. Falls Ar eine substituierte Phenylgruppe bedeutet, so ist diese vorzugsweise einfach substituiert. Sie kann jedoch auch zweifach substituiert sein, wobei die Substituenten gleich oder verschieden sein können. Bevorzugte Substituenten an der Phenylgruppe sind Methoxy und OH. Im einzelnen ist Ar bevorzugt Phenyl, o-, m- oder p-Methoxyphenyl, o-, m- oder p-Hydroxyphenyl, ferner o-, m- oder p-Ethoxyphenyl, 2,3-, 2,4-, 2,5-, 2,6-, 3,4- oder 3,5-Dimethoxyphenyl, 3-Hydroxy-4-methoxyphenyl, 3-Methoxy-4-hydroxyphenyl, 2,3-, 2,4-, 2,5-, 2,6-, 3,4- oder 3,5-Dihydroxyphenyl, 2,3- oder 3,4-Methylendioxyphenyl, 2- oder 3-Thienyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ik ausgedrückt werden, die der Formel I entsprechen und worin die nicht näher bezeichneten Reste und Parameter die bei Formel I angegebene Bedeutung haben, worin jedoch

in Ia — Ind Hydroxymethyl-indol-3-yl, Formyl-indol-3-yl, Carboxy-indol-3-yl, Methoxycarbonyl-indol-3-yl, Ethoxycarbonyl-indol-3-yl, Carbamoyl-indol-3-yl, Ethoxycarbonyl-methoxy-indol-3-yl oder Carboxymethoxy-indol-3-yl bedeutet, wobei die Substituenten vorzugsweise in 5- und/oder 6-Stellung stehen;

in Ib — Ind 4-, 5-, 6- oder 7-Hydroxymethyl-indol-3-yl, 5-, 6- oder 7-Formyl-indol-3-yl, 5-, 6- oder 7-Carboxyindol-3-yl, 5-, 6- oder 7-Methoxycarbonyl-indol-3-yl, 5-, 6- oder 7-Ethoxycarbonyl-indol-3-yl, 5-, 6- oder 7-Carbamoyl-indol-3-yl, 5-Methoxy-6-ethoxycarbonyl-indol-3-yl oder 5-Methoxy-6-carboxy-indol-3-yl bedeuten;

in Ic — A $-(CH_2)_n-$ oder $-CH_2-S-CH_2CH_2-$ bedeuten;

in Id — A $-(CH_2)_4-$ bedeutet;

in Ie — die beiden Reste Y zusammen eine C-C-Bindung bedeuten;

in If — Ar Phenyl, Hydroxyphenyl oder Methoxyphenyl bedeutet;

in Ig — Ar in 4-Stellung steht und Phenyl bedeutet;

in Ih — Ind 4-, 5-, 6- oder 7-Hydroxymethyl-indol-3-yl, 5-, 6- oder 7-Formyl-indol-3-yl, 5-, 6- oder 7-Carboxy-indol-3-yl, 5-, 6- oder 7-Methoxycarbonyl-indol-3-yl, 5-, 6- oder 7-Ethoxycarbonyl-indol-3-yl, 5-, 6- oder 7-Carbamoyl-indol-3-yl, 5-Methoxy-6-ethoxycarbonyl-indol-3-yl oder 5-Methoxy-6-carboxy-indol-3-yl,
A $-(CH_2)_n-$ oder $-CH_2-S-CH_2CH_2-$ und
Ar Phenyl, Hydroxyphenyl oder Methoxyphenyl bedeuten;

in Ii — Ind 4-, 5-, 6- oder 7-Hydroxymethyl-indol-3-yl, 5-, 6- oder 7-Formyl-indol-3-yl, 5-, 6- oder 7-Carboxy-indol-3-yl, 5-, 6- oder 7-Methoxycarbonyl-indol-3-yl, 5-, 6- oder 7-Ethoxycarbonyl-indol-3-yl, 5-, 6- oder 7-Carbamoyl-indol-3-yl, 5-Methoxy-6-ethoxycarbonyl-indol-3-yl oder 5-Methoxy-6-carboxy-indol-3-yl,
A $-(CH_2)_4-$ oder $-CH_2-S-CH_2CH_2-$ und
Ar Phenyl, m-Hydroxyphenyl oder p-Hydroxyphenyl bedeuten;

in Ij — Ind Carboxy-indol-3-yl oder Carbamoyl-indol-3-yl
A $-(CH_2)_4-$ oder $-CH_2-S-CH_2CH_2-$ und
Ar Phenyl, m-Hydroxyphenyl oder p-Hydroxyphenyl bedeuten;

in Ik — Ind 5-Carboxyindol-3-yl oder 5-Carbamoylindol-3-yl,
A $-(CH_2)_4-$ oder $-CH_2-S-CH_2CH_2-$ und die beiden Reste Y zusammen eine C-C-Bindung bedeuten

und

Ar in 4-Stellung steht und Phenyl bedeutet.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Gegenstand der Erfindung ist ferner das in Patentanspruch 3 beschriebene Verfahren zur Herstellung der Verbindungen der Formel I sowie ihrer physiologisch unbedenklichen Säureadditionssalze.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z. B. Methansulfonyloxy) oder Arylsulfonyloxy mit 6-10 C-Atomen (z. B. Benzolsulfonyloxy, p-Toluolsulfonyloxy, 1- oder 2-Naphthalin-sulfonyloxy).

Dementsprechend sind die Indolderivate der Formel I insbesondere durch Umsetzung von Verbindungen der Formel Ind-A-Cl oder Ind-A-Br mit Piperidin- bzw. Tetrahydropyridinderivaten der Formel III, worin $X^2$ und $X^3$ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden. Verbindungen der Formel II ($A=-CH_2-S-CH_2CH_2-$) können z. B. hergestellt werden aus Mannich-Basen der Formel IV und Thiolen der Formel $HS-CH_2CH_2-X^1$, z. B. $HS-CH_2CH_2OH$. Die Sulfoxide und Sulfone der Formel II ($A=-CH_2-SO-CH_2CH_2-$ oder $-CH_2-SO_2-CH_2CH_2-$) sind durch Oxydation der Thioether (II, $A=-CH_2-S-CH_2CH_2-$) zugänglich. Primäre Alkohole der Formel Ind-A-OH sind z. B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-A-Hal. Die entsprechenden Sulfonyloxyverbindungen sind erhältlich aus den Alkoholen Ind-A-OH durch Umsetzung mit den entsprechenden Sulfonsäurechloriden.

Die Jodverbindungen der Formel Ind-A-J sind z. B. durch Einwirkung von Kaliumjodid auf die zugehörigen p-Toluol-sulfonsäureester erhältlich. Die Amine der Formel $Ind-A-NH_2$ sind z. B. aus den Halogeniden mit Phthalimidkalium oder durch Reduktion der entsprechenden Nitrile herstellbar.

Die Piperidinderivate IIIa sind größtenteils bekannt (vgl. DE-OS-20 60 816) und z. B. erhältlich durch Umsetzung von 3- oder 4-Piperidon mit metallorganischen Verbindungen der Formel M-Ar (worin M ein Li-Atom oder MgHal bedeutet), anschließende Hydrolyse zu den entsprechenden 3-Ar-3-hydroxy- oder 4-Ar-4-hydroxypiperidinen sowie gewünschtenfalls nachfolgende Dehydratisierung zu 3- oder 4-Ar-3,4-dehydropiperidinen. Verbindungen der Formel III ($X^2$ und $X^3$=jeweils X) sind z. B. herstellbar durch Reduktion von Diestern der Formel $AlkylOOC-CH_2-CYZ-CYZ-COOAlkyl$ zu Diolen der Formel $HO-CH_2CH_2-CYZ-CYZ-CH_2OH$ (III, $X^2=X^3=OH$) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetoniril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente $Ind-A-NH_2$ bzw. des Piperidinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der

oben angeführten Gruppen bzw. zusätzlichen Bindungen enthalten, reduktiv in eine Verbindung der Formel I überzuführen; dabei können gleichzeitig COW-Gruppen, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner.

Bevorzugste Ausgangsstoffe für die Reduktion entsprechen der Formel VII

Ind'-L-Q VII

worin

Ind' einen durch eine Hydroxymethyl- oder COW-Gruppe substituierten Indol-3-yl-rest, der zusätzlich ein- oder zweifach durch Alkyl, O-Alkyl, OH, F, Cl, Br und/oder O-Benzyl und/oder durch eine Arylsulfonylgruppe oder eine Benzylgruppe in 1-Stellung substituiert sein kann,

L A oder eine dem Rest A entsprechende Kette, worin jedoch eine oder mehrere $-CH_2$-Gruppe(n) durch -CO- und/oder ein oder mehrere Wasserstoffatome durch OH-Gruppen ersetzt sind,

Q 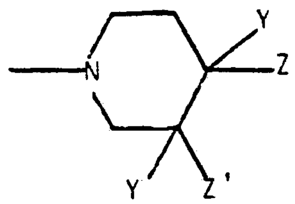 oder 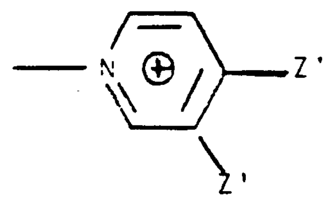 An$^{\ominus}$ ;

der eine Rest Z' Ar',

der andere Rest Z' H,

An− ein Anion einer starken Säure und

Ar' eine unsubstituierte oder ein- oder zweifach durch O-Alkyl, OH und/oder O-Benzyl oder durch eine Methylendioxygruppe substituierte Phenylgruppe bedeuten,

worin jedoch nicht gleichzeitig Ind' = Ind, L = A,

Q 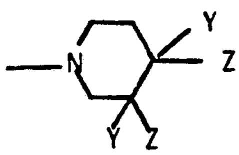 und Ar' = Ar sein können.

In den Verbindungen der Formel VII ist L bevorzugt $-CO-(CH_2)_{n-2}-CO-$ [im einzelnen -COCO-, $-COCH_2CO-$, $-CO-(CH_2)_2-CO-$, $-CO-(CH_2)_3-CO-$], $-(CH_2)_{n-1}-CO-$ [im einzelnen $-CH_2CO-$, $-CH_2CH_2-CO-$, $-(CH_2)_3-CO-$ oder $-(CH_2)_4-CO-$], $-CH_2-S-CH_2-CO-$, $-CH_2-SO-CH_2-CO-$ oder $-CH_2-SO_2-CH_2-CO-$, ferner z. B. $-CO-CH_2CH_2-$, $-CH_2-CO-CH_2-$, $-CO-(CH_2)_3-$, $-CH_2-CO-CH_2CH_2-$, $-CH_2CH_2-CO-CH_2-$, $-CO-(CH_2)_4-$, $-CH_2-CO-(CH_2)_3-$, $-CH_2CH_2-CO-CH_2CH_2-$ oder $-(CH_2)_3-CO-CH_2-$.

Verbindungen der Formel VII sind z. B. herstellbar durch Umsetzung von 4-Ar'-1,2,3,6-tetrahydropyridin oder 4-Ar'-pyridin mit einer Verbindung der Formel VIII

Ind'-L-$X^1$ VIII

worin

Ar', Ind', L und $X^1$ die oben angegebenen Bedeutungen haben,

unter den Bedingungen, die oben für die Umsetzung von II mit III angegeben sind.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerigalkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxy-ethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden oder vinylogen Säureamiden (z. B. solchen

der Formel VII, worin L eine $-(CH_2)_{n-1}-CO-$, $-CH_2-S-CH_2-CO-$ oder $-CO-(CH_2)_{n-2}-CO$-Gruppe bedeutet) mit $LiAlH_4$ in THF bei Temperaturen zwischen etwa 0 und 66° zu $CH_2$-Gruppen reduzieren. Dabei können in 1-Stellung des Indolring befindliche Arylsulfonyl-Schutzgruppen gleichzeitig reduktiv abgespalten und/oder am Indolring befindliche COW-Gruppen reduziert werden, z. B. COOAlkyl-, COOH- oder CHO-Gruppen zu $CH_2OH$-Gruppen.

Eine Reduktion der Pyridiniumsalze der Formel VII

(worin Q — 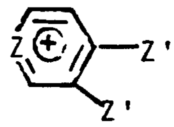 An⁻ und An

vorzugsweise Cl, Br oder $CH_3SO_3$ bedeutet) zu Verbindungen der Formel I gelingt z. B. mit $NaBH_4$ in Wasser, Methanol oder Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z. B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II ($X^1 = X$) entsprechen, aber an Stelle eines oder mehrerer H-Atome eine oder mehrere solvolysierbare Gruppe(n) enthalten. So können insbesondere 4-, 5-, 6- oder 7-Cyanindolderivate zu den entsprechenden 4-, 5-, 6- oder 7-Carbamoylindolderivaten oder 4-, 5-, 6- oder 7-Carboxyindolderivaten der Formel I oder 1-Acylindolderivate (entsprechend der Formel I, aber in 1-Stellung des Ind-Rests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z. B. in saurem, besser in neutralem oder alkalischem Medium bei Temperaturen zwischen 0 und 200°.

Als basische Katalysatoren verwendet man zweckmäßig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Indolderivate der Formel I ($A = -CH_2-S-CH_2CH_2-$) sind ferner durch Umsetzung von Mannich-Basen der Formel IV mit Thiolen der Formel V (oder ihren Salzen) erhältlich.

Die Ausgangsstoffe der Formeln IV und V sind teilweise bekannt; die nicht bekannten unter diesen Ausgangsstoffen können leicht analog zu den bekannten Verbindungen hergestellt werden. So sind die Mannich-Basen der Formel IV z. B. aus Indolen der Formel Ind-H, Formaldehyd und Aminen der Formel $HN(R)_2$ erhältlich, die Thiole der Formel V aus den Basen der Formel IIIa und Thiolderivaten der Formel $HS-CH_2CH_2-X^1$ (wobei die HS-Gruppe auch intermediär geschützt werden kann).

Im einzelnen erfolgt die Umsetzung IV mit V in Gegenwart oder Abwesenheit eines inerten Lösungsmittels bei Temperaturen zwischen etwa -20 und 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin; Pyridin oder Picoline; Alkohole wie Methanol, Ethanol oder Butanol; Glykole und Glykolether wie Ethylenglykol, Diethylenglykol, 2-Methoxy-ethanol; Ketone wie Aceton; Ether wie THF oder Dioxan; Amide wie DMF; Sulfoxide wie Dimethylsulfoxid. Auch Gemische dieser Lösungsmittel sind geeignet. Die Thiole der Formel V werden zweckmäßig zunächst in die entsprechenden Mercaptide umgewandelt, vorzugsweise durch Reaktion mit Natrium- oder Kaliumhydroxid, Natrium- oder Kaliumethylat in die entsprechenden Natrium- oder Kaliummercaptide.

Man gelangt ferner zu Verbindungen der Formel I, indem man aus Verbindungen der Formel VI unter Ausbildung einer Doppelbindung HE abspaltet. Entsprechend der Definition von E kann es sich z. B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel VI sind z. B. erhältlich durch Umsetzung von II ($X^1 = X$) mit einer Verbindung der Formel IX

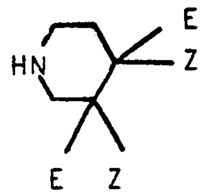

IX

worin E und Z die angegebenen Bedeutungen haben.

Falls einer der Reste E=Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z. B. Kalium-tert.-butylat, Amine, wie z. B. Dimethylanilin, Pyridin, Collidin oder Chinolin; als Lösungsmittel benutzt man z. B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z. B. Wasser oder Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z. B. die der Mesylate oder Tosylate, erfolgt schonend durch Kochen in DMF oder Dimethylsulfoxid mit Alkalimetallcarbonaten, z. B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel VI (eine Gruppe E=CN) durch Erhitzen abgespalten werden. Die Eliminierung von HE aus VI erfolgt allgemein bei Temperaturen zwischen 0 und etwa 250°, vorzugsweise zwischen 50 und 200°.

Weiterhin kann man gegebenenfalls eine Verbindung der Formel I nach an sich bekannten Methoden in eine andere Verbindung der Formel I umwandeln.

So kann man in einem Thioether der Formel I (A=$-CH_2-S-CH_2CH_2-$) die Thioethergruppe zu einer SO-Gruppe oder zu einer $SO_2$-Gruppe oder in einem Sulfoxid der Formel I (A=$-CH_2-SO-CH_2CH_2-$) die SO-Gruppe zu einer $SO_2$-Gruppe oxydieren. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°). Will man dagegen die Sulfone (aus den Thioethern oder den Sulfoxiden) erhalten, so verwendet man die gleichen Oxydationsmittel unter kräftigeien Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$. Ein bevorzugtes Oxydationsmittel ist 30 %-iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100° zu den Sulfoxiden, im Überschuß bei höheren Temperaturen, vorzugsweise in Essigsäure oder in einem Gemisch aus Essigsäure und Acetanhydrid, zu den Sulfonen.

Ether der Formel I, in denen die Reste Ind und/oder Ar ein- oder zweifach durch O-Alkyl substituiert sind, können gespalten werden, wobei die entsprechenden Hydroxyderivate entstehen. Z. B. kann man die Ether spalten durch Behandeln mit Dimethylsulfid-Bortribromid-Komplex, z. B. in Toluol, Ethern wie THF oder Dimethylsulfoxid, oder durch Verschmelzen mit Pyridin- oder Anilin-hydrohalogeniden, vorzugsweise Pyridinhydrochlorid, bei etwa 150-250°, oder durch Behandeln mit Diisobutylaluminiumhydrid in Toluol bei etwa 0-110°.

Weiterhin kann man nach an sich bekannter Methoden COW-Gruppen in andere COW-Gruppen umwandeln. So ist es möglich, Aldehydgruppen zu Carboxylgruppen zu oxydieren, beispielsweise mit $MnO_2$ in einem inerten Lösungsmittel wie Dichlormethan. Andererseits kann man Carboxylgruppen reduzieren, z. B. mit Diisobutylaluminiumhydrid in Toluol. Carboxylgruppen können verestert werden, z. B. durch Behandeln mit Alkoholen in Gegenwart eines sauren Katalysators oder durch Reaktion mit Diazoalkanen. Umwandlung der Carbonsäuren in ihre Chloride, z. B. mit $SOCl_2$, und nachfolgende Reaktion mit $NH_3$ oder Aminen führt zu den entsprechenden Carboxamiden, die auch durch Behandeln der Carbonsäureester mit Ammoniak oder Aminen erhältlich sind. Eine Solvolyse, vorzugsweise eine Hydrolyse unter den oben angegebenen Bedingungen, führt von den Estern oder Amiden zu den Carbonsäuren; insbesondere sind Carbonsäuren aus den Carbamoylverbindungen erhältlich, indem man diese mit NaOH oder KOH in wässerigen Glykolen oder Glykolethern, z. B. Diethylenglykolmonomethyl- oder -ethylether behandelt, zweckmäßig bei Temperaturen zwischen etwa 50 und etwa 200°.

Eine Reduktion von COW-, insbesondere Formyl-, Alkoxycarbonyl- oder Carboxylgruppen kann auch zu Hydroxymethylgruppen führen. Zweckmäßig verwendet man ein komplexes Hydrid wie $LiAlH_4$; Aldehyde und Ester können auch mit anderen der oben aufgeführten Reduktionsmittel reduziert werden. Vorzugsweise

arbeitet man unter den oben angegebenen Bedingungen. Umgekehrt kann man Hydroxymethylgruppen, z. B. mit $MnO_2$ oder $CrO_3$ oder deren Derivaten, zu Formyl- oder Carboxylgruppen oxydieren.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline®. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können verwendet werden bei der therapeutischen Behandlung des menschlichen oder tierischen Köpers und bei der Bekämpfung von Krankheiten, insbesondere von Parkinsonismus, von extrapyramidalen Störungen bei der Neuroleptikatherapie, von Depressionen und/oder Psychosen und von Nebenwirkungen bei der Behandlung der Hypertonie (z. B. mit α-Methyldopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z. B. Migräne), insbesondere in der Geriatrie ähnlich wie gewisse Ergot-Alkaloide, sowie auch zur Blutdrucksenkung.

Dabei werden die erfindungsgemäßen Substanzen in der Regel in Analogie zu bekannten, im Handel befindlichen Präparaten (z. B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkte und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

**Beispiel 1**

Man rührt eine Lösung von 28,4 g 3-(4-Chlor-2-thiabutyl)-indol-5-carbonsäuremethylester [oder 32,8 g 3-(4-Brom-2-thiabutyl)-indol-5-carbonsäuremethylester; erhältlich durch Reaktion von Gramin-5-carbonsäuremethylester mit 2-Mercaptoethanol zu 3-(4-Hydroxy-2-thiabutyl)-indol-5-carbonsäuremethylester und nachfolgende Umsetzung mit $SOCl_2$ oder $PBr_3$] und 16 g 4-Phenyl-1,2,3,6-tetrahydropyridin in 100 ml Acetonitril 12 Std. bei 20°, arbeitet wie üblich auf und erhält 3-[4-(4-Phenyl-1,2,3,6-tetrahydropyridyl)-2-thiabutyl]-indol-5-carbonsäuremethylester ("P"); Hydrochlorid, F. 202-203°.

Analog erhält man aus den entsprechenden Ausgangsstoffen der Formeln II und III:

3-[4-(4-Phenyl-1,2,3,6-tetrahydropyridyl)-butyl]-5-hydroxymethylindol, F. 178°
3-[4-(4-Phenyl-1,2,3,6-tetrahydropyridyl)-2-thiabutyl]-4-hydroxymethylindol
3-[4-(4-Phenyl-1,2,3,6-tetrahydropyridyl)-2-thiabutyl]-5-hydroxymethylindol, F. 118-120°
3-[4-(4-Phenyl-1,2,3,6-tetrahydropyridyl)-2-thiabutyl]-6-hydroxymethylindol, F. 142-143°
3-[4-(4-Phenyl-1,2,3,6-tetrahydropyridyl)-2-thiabutyl]-7-hydroxymethylindol
3-[4-(4-Phenyl-1,2,3,6-tetrahydropyridyl)-2-thiabutyl]-4-methoxycarbonylindol
3-[4-(4-Phenyl-1,2,3,6-tetrahydropyridyl)-2-thiabutyl]-6-methoxycarbonylindol
3-[4-(4-Phenyl-1,2,3,6-tetrahydropyridyl)-2-thiabutyl]-7-methoxycarbonylindol
3-[4-(4-Phenvl-1,2,3,6-tetrahydropyridyl)-2-thiabutyl]-4-ethoxycarbonylindol
3-[4-(4-Phenyl-1,2,3,6-tetrahydropyridyl)-2-thiabutyl]-5-ethoxycarbonylindol
3-[4-(4-Phenyl-1,2,3,6-tetrahydropyridyl)-2-thiabutyl]-6-ethoxycarbonylindol, F. 127-129°
3-[4-(4-Phenyl-1,2,3,6-tetrahydropyridyl)-2-thiabutyl]-7-ethoxycarbonylindol.

**Beispiel 2**

Ein Gemisch von 2,64 g 3-(4-Amino-2-thiabutyl)-indol-5-carbonsäuremethylester [erhältlich durch Reaktion von 3-(4-Brom-2-thiabutyl)-indol-5-carbonsäuremethylester mit Phthalimidkalium und anschließende Hydrolyse] und 2,15 g 1,5-Dichlor-3-phenyl-2-penten (erhältlich durch Reduktion von 3-Phenyl-2-penten-1,5-disäurediethylester mit $LiAlH_4$ und anschließende Reaktion mit $SOCl_2$) in 40 ml Aceton und 40 ml Wasser wird 24 Stunden gekocht und wie üblich aufgearbeitet. Man erhält "P", Hydrochlorid, F. 202-203°.

**Beispiel 3**

Zu einer Suspension von 23,4 g $LiAlH_4$ in 1100 ml absolutem THF tropft man unter Rühren eine Suspension von 41,6 g 3-[4-(4-Phenyl-1,2,3,6-tetrahydropyridyl)-1,4-dioxobutyl]-indol-5-carbonsäuremethylesrer (F. 218°; erhältlich aus 4-(5-Methoxycarbonyl-3-indol)-4-oxobuttersäure und 4-Phenyl-1,2,3,6-tetrahydropyridin) in 3 l heißem absolutem THF, kocht 1 Std., kühlt ab, zersetzt mit Wasser und Natronlauge und arbeitet wie üblich auf. Man erhält 3-[4-(4-Phenyl-1,2,3,6-tetrahydropyridyl)-butyl]-5-hydroxymethylindol, F. 178°.

Analog sind aus den entsprechenden Dioxoestern, z. B.

3-[4-(4-Phenyl-1,2,3,6-tetrahydropyridyl)-1,4-dioxobutyl]-indol-4-carbonsäuremethylester, F. 228°
3-[4-(4-Phenyl-1,2,3,6-tetrahydropyridyl)-1,4-dioxobutyl]-indol-6-carbonsäuremethylester, F. 237°
3-[4-(4-Phenyl-1,2,3,6-tetrahydropyridyl)-1,4-dioxobutyl]-indol-7-carbonsäuremethylester, F. 208°

erhältlich:

3-[4-(4-Phenyl-1,2,3,6-tetrahydropyridyl)-butyl]-4-hydroxymethylindol, F. 183-184°
3-[4-(4-Phenyl-1,2,3,6-tetrahydropyridyl)-butyl]-6-hydroxymethylindol, F. 179°
3-[4-(4-Phenyl-1,2,3,6-tetrahydropyridyl)-butyl]-7-hydroxymethylindol, F. 178°.

Analog erhält man aus 3-[4-Oxo-4-(4-phenyl-1,2,3,6-tetrahydropyridyl)-butyl]-indol-2-carbonsäure mit $LiAlH_4$ in THF:

3-[4-(4-Phenyl-1,2,3,6-tetrahydropyridyl)-butyl]-indol-2-carbonsäure, F. 206-208°.

**Beispiel 4**

Zu einer Lösung von 4,51 g 1-[4-(5-Carboxy-3-indolyl)-butyl]-4-phenylpyridinium-bromid [erhältlich aus 3-(4-Brombutyl)-indol-5-carbonsäure und 4-Phenyl-pyridin] in 50 ml In NaOH gibt man unter Rühren 1 g $NaBH_4$ in 20 ml Wasser und rührt danach noch 3 Stunden bei 60°. Nach üblicher Aufarbeitung erhält man 3-[4-(4-Phenyl-1,2,3,6-tetrahydropyridyl)-butyl]-indol-5-carbonsäure, F. 284-285°.

**Beispiel 5**

Ein Gemisch von 35,5 g 3-[4-(4-Phenyl-1,2,3,6-tetrahydropyridyl)-butyl]-5-cyan-indol [F. 167°; erhältlich aus der entsprechenden 5-Formylverbindung über das Oxim], 27,1 g NaOH, 520 ml Wasser und 420 ml Diethylenglykolmonoethylether wird 3 Std. bei 140° Badtemperatur gerührt. Man kühlt ab, arbeitet wie üblich auf und erhält 3-[4-(4-Phenyl-1,2,3,6-tetrahydropyridyl)-butyl]-indol-5-carboxamid, F. 200-205°.

Analog erhält man durch Hydrolyse der entsprechenden Nitrile:
3-[2-(4-Phenyl-1,2,3,6-tetrahydropyridyl)-ethyl]-indol-5-carboxamid
3-[3-(4-Phenyl-1,2,3,6-tetrahydropyridyl)-propyl]-indol-5-carboxamid
3-[4-(4-Phenyl-1,2,3,6-tetrahydropyridyl)-butyl]-indol-4-carboxamid
3-[4-(4-Phenyl-1,2,3,6-tetrahydropyridyl)-butyl]-indol-6-carboxamid, F. 226°
3-[4-(4-Phenyl-1,2,3,6-tetrahydropyridyl)-butyl]-indol-7-carboxamid, F. 203°
3-[4-(4-Phenyl-1,2,3,6-tetrahydropyridyl)-butyl]-5-methoxyindol-6-carboxamid
3-[4-(4-Phenyl-1,2,3,6-tetrahydropyridyl)-butyl]-5-hydroxyindol-6-carboxamid
3-[4-(4-Phenyl-1,2,3,6-tetrahydropyridyl)-butyl]-7-chlorindol-4-carboxamid
3-[4-(4-m-Hydroxyphenyl-1,2,3,6-tetrahydropyridyl)-butyl]-indol-5-carboxamid
3-[4-(4-p-Hydroxyphenyl-1,2,3,6-tetrahydropyridyl)-butyl]-indol-5-carboxamid
3-[5-(4-Phenyl-1,2,3,6-tetrahydropyridyl)-pentyl]-indol-5-carboxamid
3-[4-(3-Phenyl-1,2,3,6-tetrahydropyridyl)-butyl]-indol-5-carboxamid
3-[4-(3-m-Hydroxyphenyl-1,2,3,6-tetrahydropyridyl)-butyl]-indol-5-carboxamid
3-[4-(3-m-Hydroxyphenyl-1,2,3,6-tetrahydropyridyl)-butyl]-indol-6-carboxamid
3-[4-(3-p-Hydroxyphenyl-1,2,3,6-tetrahydropyridyl)-butyl]-indol-5-carboxamid
3-[4-(3-p-Hydroxyphenyl-1,2,3,6-tetrahydropyridyl)-butyl]-indol-6-carboxamid
3-[4-(4-Phenyl-1,2,3,6-tetrahydropyridyl)-2-thiabutyl]-indol-4-carboxamid
3-[4-(4-Phenyl-1,2,3,6-tetrahydropyridyl)-2-thiabutyl]-indol-5-carboxamid
3-[4-(4-Phenyl-1,2,3,6-tetrahydropyridyl)-2-thiabutyl]-indol-6-carboxamid
3-[4-(4-Phenyl-1,2,3,6-tetrahydropyridyl)-2-thiabutyl]-indol-7-carboxamid
3-[4-(4-Phenyl-1,2,3,6-tetrahydropyridyl)-2-thiabutyl]-5-methoxyindol-6-carboxamid
3-[4-(4-Phenyl-1,2,3,6-tetrahydropyridyl)-2-thiabutyi]-5-hydroxyindol-6-carboxamid
3-[4-(4-Phenyl-1,2,3,6-tetrahydropyridyl)-2-thiabutyl]-7-chlorindol-4-carboxamid
3-[4-(4-m-Hydroxyphenyl-1,2,3,6-tetrahydropyridyl]-2-thiabutyl]-indol-5-carboxamid
3-[4-(4-p-Hydroxyphenyl-1,2,3,6-tetrahydropyridyl)-2-thiabutyl]-indol-5-carboxamid
3-[4-(3-m-Hydroxyphenyl-1,2,3,6-tetrahydropyridyl)-2-thiabutyl]-indol-5-carboxamid
3-[4-(3-m-Hydroxyphenyl-1,2,3,6-tetrahydropyridyl)-2-thiabutyl]-indol-6-carboxamid
3-[4-(3-p-Hydroxyphenyl-1,2,3,6-tetrahydropyridyl)-2-thiabutyl]-indol-5-carboxamid
3-[4-(3-p-Hydroxyphenyl-1,2,3,6-tetrahydropyridyl)-2-thiabutyl]-indol-6-carboxamid
3-[4-(4-Phenylpiperidino)-butyl]-indol-5-carboxamid
3-[4-(4-Phenylpiperidino)-butyl]-indol-6-carboxamid
3-[4-(4-m-Hydroxyphenylpiperidino)-butyl]-indol-5-carboxamid
3-[4-(4-m-Hydroxyphenylpiperidino)-butyl]-indol-6-carboxamid
3-[4-(4-p-Hydroxyphenylpiperidino)-butyl]-indol-5-carboxamid
3-[4-(4-p-Hydroxyphenylpiperidino)-butyl]-indol-6-carboxamid
3-[4-(3-m-Hydroxyphenylpiperidino)-butyl]-indol-5-carboxamid
3-[4-(3-m-Hydroxyphenylpiperidino)-butyl]-indol-6-carboxamid
3-[4-(3-p-Hydroxyphenylpiperidino)-butyl]-indol-5-carboxamid
3-[4-(3-p-Hydroxyphenylpiperidino)-butyl]-indol-6-carboxamid
3-[4-(4-Phenylpiperidino)-2-thiabutyl]-indol-5-carboxamid
3-[4-(4-Phenylpiperidino)-2-thiabutyl)-indol-6-carboxamid
3-[4-(4-m-Hydroxyphenylpiperidino)-2-thiabutyl]-indol-5-carboxamid
3-[4-(4-m-Hydroxyphenylpiperidino)-2-thiabutyl)-indol-6-carboxamid
3-[4-(4-p-Hydroxyphenylpiperidino)-2-thiabutyl]-indol-5-carboxamid
3-[4-(4-p-Hydroxyphenylpiperidino)-2-thiabutyl]-indol-6-carboxamid
3-[4-(3-m-Hydroxyphenylpiperidino)-2-thiabutyl]-indol-5-carboxamid
3-[4-(3-m-Hydroxyphenylpiperidino)-2-thiabutyl]-indol-6-carboxamid
3-[4-(3-p-Hydroxyphenylpiperidino)-2-thiabutyl]-indol-5-carboxamid
3-[4-(3-p-Hydroxyphenylpiperidino)-2-thiabutyl]-indol-6-carboxamid.

**Beispiel 6**

Man arbeitet wie in Beispiel 5 beschrieben, kocht aber 16 Std. und erhält nach üblicher Aufarbeitung 3-[4-(4-Phenyl-1,2,3,6-tetrahydropyridyl)-butyl]-indol-5-carbonsäure, F. 284-285°.

Analog erhält man durch Hydrolyse der entsprechenden Nitrile:
3-[2-(4-Phenyl-1,2,3,6-tetrahydropyridyl)-ethyl]-indol-5-carbonsäure

3-[3-(4-Phenyl-1,2,3,6-tetrahydropyridyl)-propyl]-indol-5-carbonsäure
3-[4-(4-Phenyl-1,2,3,6-tetrahydropyridyl)-butyl]-indol-4-carbonsäure
3-[4-(4-Phenyl-1,2,3,6-tetrahydropyridyl)-butyl]-indol-6-carbonsäure, F. 268°
3-[4-(4-Phenyl-1,2,3,6-tetrahydropyridyl)-butyl]-indol-7-carbonsäure, F. 262-265°
3-[4-(4-Phenyl-1,2,3,6-tetrahydropyridyl)-butyl]-5-methoxyindol-6-carbonsäure
3-[4-(4-Phenyl-1,2,3,6-tetrahydropyridyl)-butyl]-5-hydroxyindol-6-carbonsäure
3-[4-(4-Phenyl-1,2,3,6-tetrahydropyridyl)-butyl)-7-chlorindol-4-carbonsäure, F. 263-266°
3-[4-(4-o-Methoxyphenyl-1,2,3,6-tetrahydropyridyl)-butyl]-indol-5-carbonsäure
3-[4-(4-m-Methoxyphenyl-1,2,3,6-tetrahydropyridyl)-butyl]-indol-5-carbonsäure
3-[4-(4-p-Methoxyphenyl-1,2,3,6-tetrahydropyridyl)-butyl]-indol-5-carbonsäure
3-[4-(4-o-Hydroxyphenyl-1,2,3,6-tetrahydropyridyl)-butyl]-indol-5-carbonsäure
3-[4-(4-m-Hydroxyphenyl-1,2,3,6-tetrahydropyridyl)-butyl]-indol-5-carbonsäure
3-[4-(4-p-Hydroxyphenyl-1,2,3,6-tetrahydropyridyl)-butyl]-indol-5-carbonsäure
3-[4-(4-(3-Methoxy-4-hydroxyphenyl)-1,2,3,6-tetrahydropyridyl)-butyl]-indol-5-carbonsäure
3-[4-(4-(3,4-Dimethoxyphenyl)-1,2,3,6-tetrahydropyridyl)-butyl]-indol-5-carbonsäure
3-[4-(4-(3,4-Methylendioxyphenyl)-1,2,3,6-tetrahydropyridyl)-butyl]-indol-5-carbonsäure
3-[4-(4-(2-Thienyl)-1,2,3,6-tetrahydropyridyl)-butyl]-indol-5-carbonsäure
3-[4-(4-(3-Thienyl)-1,2,3,6-tetrahydropyridyl)-butyl]-indol-5-carbonsäure
3-[5-(4-Phenyl-1,2,3,6-tetrahydropyridyl)-pentyl]-indol-5-carbonsäure
3-[4-(3-Phenyl-1,2,3,6-tetrahydropyridyl)-butyl]-indol-5-carbonsäure
3-[4-(3-p-Methoxyphenyl-1,2,3,6-tetrahydropyridyl)-butyl]-indol-5-carbonsäure
3-[4-(3-m-Hydroxyphenyl-1,2,3,6-tetrahydropyridyl)-butyl]-indol-5-carbonsäure
3-[4-(3-m-Hydroxyphenyl-1,2,3,6-tetrahydropyridyl)-butyl]-indol-6-carbonsäure
3-[4-(3-p-Hydroxyphenyl-1,2,3,6-tetrahydropyridyl)-butyl]-indol-5-carbonsäure
3-[4-(3-Hydroxyphenyl-1,2,3,6-tetrahydropyridyl)-butyl]-indol-6-carbonsäure
3-[4-(4-Phenylpiperidino)-butyl]-indol-5-carbonsäure
3-[4-(4-Phenylpiperidino)-butyl]-indol-6-carbonsäure
3-[4-(4-m-Hydroxyphenylpiperidino)-butyl]-indol-5-carbonsäure
3-[4-(4-m-Hydroxyphenylpiperidino)-butyl]-indol-6-carbonsäure
3-[4-(4-p-Hydroxyphenylpiperidino)-butyl]-indol-5-carbonsäure
3-[4-(4-p-Hydroxyphenylpiperidino)-butyl]-indol-6-carbonsäure
3-[4-(3-m-Hydroxyphenylpiperidino)-butyl]-indol-5-carbonsäure
3-[4-(3-m-Hydroxyphenylpiperidino)-butyl]-indol-6-carbonsäure
3-[4-(3-p-Hydroxyphenylpiperidino)-butyl]-indol-5-carbonsäure
3-[4-(3-p-Hydroxyphenylpiperidino)-butyl]-indol-6-carbonsäure
3-[4-(4-Phenylpiperidino)-butyl]-7-chlorindol-4-carbonsäure
3-[4-(3-m-Hydroxyphenylpiperidino)-butyl]-7-chlorindol-4-carbonsäure.

**Beispiel 7**

Man kocht 4,68 g 1-Benzolsulfonyl-3-[4-(4-Phenyl-1,2,3,6-tetrahydropyridyl)-butyl]-indol-5-carbonsäuremethylester [erhältlich aus 1-Benzolsulfonyl-3-(4-chlorbutyl)-indo-5-carbonsäuremethylester und 4-Phenyl-1,2,3,6-tetrahydro-pyridin] mit 1 g KOH in 7 ml Wasser und 14 ml Ethanol 16 Stunden, konzentriert das Gemisch, arbeitet wie üblich auf und erhält 3-[4-(4-Phenyl-1,2,3,6-tetrahydropyridyl)-butyl]-indol-5-carbonsäure, F. 284-285°.

**Beispiel 8**

Man löst 2,76 g Na in 180 ml Ethanol, gibt 21,9 g 1-(2-Mercaptoethyl)-4-phenyl-1,2,3,6-tetrahydropyridin [erhältlich durch Reaktion von 4-Phenyl-1,2,3,6-tetrahydropyridin mit Thioglykolsäure zu 1-(2-Mercaptoacetyl)-4-phenyl-1,2,3,6-tetrahydropyridin und Reduktion mit $LiAlH_4$] und 23,2 g Gramin-5-carbonsäuremethylester hinzu, kocht 16 Stunden, dampft ein, arbeitet wie üblich auf und erhält "P", Hydrochlorid, F. 202-203°.

Analog erhält man aus den entsprechenden Ausgangsstoffen der Formeln IV und V:

3-[4-(4-Phenyl-1,2,3,6-tetrahydropyridyl)-2-thiabutyl]-5-methoxyindol-6-carbonsäuremethylester
3-[4-(4-Phenyl-1,2,3,6-tetrahydropyridyl)-2-thiabutyl]-5-methoxyindol-6-carbonsäureethylester, Hydrochlorid, F. 169-173°
3-[4-(4-Phenyl-1,2,3,6-tetrahydropyridyl)-2-thiabutyl]-5-hydroxyindol-6-carbonsäuremethylester
3-[4-(4-Phenyl-1,2,3,6-tetrahydropyridyl)-2-thiabutyl]-5-hydroxyindol-6-carbonsäureethylester
3-[4-(4-Phenyl-1,2,3,6-tetrahydropyridyl)-2-thiabutyl]-7-chlorindol-4-carbonsäuremethylester.

**Beispiel 9**

Man erhitzt 4,05 g 1-Methyl-3-[4-(4-hydroxy-4-phenyl-1-piperidyl)-butyl]-indol-5-carboxamid [erhältlich durch Reaktion von 1-Methyl-3-(4-brombutyl)-indol-5-carboxamid mit 4-Piperidon, anschließende Umsetzung mit $C_6H_5Li$ und Hydrolyse] mit 40 ml Salzsäure 2 Stunden auf 50°, arbeitet wie üblich auf und erhält 1-Methyl-3-[4-(4-phenyl-1,2,3,6-tetrahydropyridyl)-butyl]-indol-5-carboxamid.

**Beispiel 10**

Zu einer siedenden Lösung von 4,06 g "P" in 50 ml Ethanol gibt man 6 ml 30 %-iges $H_2O_2$ und kocht anschließend 3 Stunden. Nach Zugabe weiterer 4 ml des Oxydationsmittels kocht man noch 9 Stunden, kühlt ab, arbeitet wie üblich auf und erhält 3-[4-(4-Phenyl-1,2,3,6-tetrahydropyridyl)-2-thiabutyl]-indol-5-carbonsäuremethylester-S-oxid.

**Beispiel 11**

Zu einer Lösung von 4,06 g "P" in 20 ml Essigsäure gibt man 9 ml 30 %-iges $H_2O_2$ und kocht 90 Minuten. Nach der üblichen Aufarbeitung erhält man 3-[4-(4-Phenyl-1,2,3,6-tetrahydropyridyl)-2-thiabutyl]-indol-5-carbonsäuremethylester-S,S-dioxid.

**Beispiel 12**

Ein Gemisch von 4,04 g 3-[4-(4-p-Methoxyphenyl-1,2,3,6-tetrahydropyridyl)-butyl]-indol-5-carbonsäure und 3,5 g Pyridinhydrochlorid wird 3 Stunden bei 160° gerührt. Nach üblicher Aufarbeitung erhält man 3-[4-(4-p-Hydroxyphenyl-1,2,3,6-tetrahydropyridyl)-butyl]-indol-5-carbonsäure.

**Beispiel 13**

Man löst 36 g 3-[4-(4-Phenyl-1,2,3,6-tetrahydropyridyl)-butyl]-5-hydroxymethylindol in 1,6 l THF und gibt 300 ml Ether hinzu. Unter Rühren werden 55 g $MnO_2$ zugegeben. Man rührt 16 Std. bei 20°, gibt portionsweise weitere 100 g $MnO_2$ hinzu und rührt weitere 100 Std. bei 20°. Nach Filtration und üblichem Aufarbeiten erhält man 3-[4-(4-Phenyl-1,2,3,6-tetrahydropyridyl)-butyl]-5-formylindol, F. 131°.

Analog erhält man durch Oxydation der entsprechenden Hydroxymethylindole:

3-[4-(4-Phenyl-1,2,3,6-tetrahydropyridyl)-butyl]-2-formylindol, F. 129-130°.
3-[4-(4-Phenyl-1,2,3,6-tetrahydropyridyl)-butyl]-4-formylindol
3-[4-(4-Phenyl-1,2,3,6-tetrahydropyridyl)-butyl]-6-formylindol
3-[4-(4-Phenyl-1,2,3,6-tetrahydropyridyl)-butyl]-7-formylindol
3-[4-(4-Phenyl-1,2,3,6-tetrahydropyridyl)-2-thiabutyl]-4-formylindol
3-[4-(4-Phenyl-1,2,3,6-tetrahydropyridyl)-2-thiabutyl]-5-formylindol
3-[4-(4-Phenyl-1,2,3,6-tetrahydropyridyl)-2-thiabutyl]-7-formylindol.

**Beispiel 14**

Man löst 3,9 g 3-[4-(4-Phenyl-1,2,3,6-tetrahydropyridyl)-butyl]-6-hydroxymethylindol in 50 ml Dichlormethan, versetzt die Lösung mit 9 g $MnO_2$, rührt 60 Std. bei 40° und filtriert die unlöslichen Anteile ab. Nach üblicher Aufarbeitung des Filtrats erhält man 3-[4-(4-Phenyl-1,2,3,6-tetrahydropyridyl)-butyl]-indol-6-carbonsäure, F. 268°.

**Beispiel 15**

Man löst 3,88 g 3-[4-(4-Phenyl-1,2,3,6-tetrahydropyridyl)-butyl]-5-formylindol in 80 ml Dichlormethan, versetzt mit 9 g $MnO_2$ und rührt die Suspension 48 h bei 40°. Nach Filtrieren und üblicher Aufarbeitung erhält man 3-[4-(4-Phenyl-1,2,3,6-tetrahydropyridyl)-butyl]-indol-5-carbonsäure, F. 284-285°.

**Beispiel 16**

Man suspendiert 4,04 g 3-[4-(4-Phenyl-1,2,3,6-tetrahydropyridyl)-butyl]-indol-5-carbonsäure in 25 ml Toluol und tropft unter $N_2$ und Rühren die 3-fach molare Menge einer 20 %-igen Lösung von Diisobutylaluminiumhydrid in Toluol zu, kocht 2 Std., kühlt ab, zersetzt das Gemisch mit Wasser, arbeitet wie üblich auf und erhält
3-[4-(4-Phenyl-1,2,3,6-tetrahydropyridyl)-butyl]-5-formylindol, F. 131°.

**Beispiel 17**

Zu einer Suspension von 0,76 g Lithiumaluminiumhydrid in 30 ml THF wird unter Rühren und $N_2$ eine Lösung von 4,04 g 3-[4-(4-Phenyl-1,2,3,6-tetrahydropyridyl)-butyl]-indol-5-carbonsäure in 40 ml THF zugetropft. Man rührt noch 2 Std. bei 20°, zersetzt mit verdünnter Natronlauge, dann mit Wasser und filtriert. Das Filtrat wird wie üblich aufgearbeitet. Man erhält 3-[4-(4-Phenyl-1,2,3,6-tetrahydropyridyl)-butyl]-5-hydroxymethylindol, F. 178°.

**Beispiel 18**

Zu einer Suspension von 0,57 g Lithiumaluminiumhydrid in 20 ml THF wird unter Rühren und $N_2$ bei 20° eine Lösung von 4,18 g 3-[4-(4-Phenyl-1,2,3,6-tetrahydropyridyl)-butyl]-indol-5-carbonsäuremethylester in 40 ml THF zugetropft. Man rührt 1 Std. bei 20°, zersetzt mit verdünnter Natronlauge, dann mit Wasser, filtriert, arbeitet das Filtrat wie üblich auf und erhält 3-[4-(4-Phenyl-1,2,3,6-tetrahydropyridyl)-butyl]-5-hydroxymethylindol, F. 178°.

Analog erhält man aus den entsprechenden Estern die in Beispiel 3 genannten Hydroxymethylverbindungen sowie 2-Hydroxymethyl-3-[4-(4-phenyl-1,2,3,6-tetrahydropyridyl)-butyl]-indol, F. 162-163,5°.

**Beispiel 19**

Zu einer Suspension von 0,57 g Lithiumaluminiumhydrid in 20 ml THF wird unter $N_2$ und Rühren eine Lösung von 3,88 g 3-[4-(4-Phenyl-1,2,3,6-tetrahydropyridyl)-butyl]-5-formylindol in 40 ml THF zugetropft. Man rührt noch 1 Std. bei 20°, zersetzt mit verdünnter Natronlauge, dann mit Wasser, filtriert ab, arbeitet wie üblich auf und erhält 3-[4-(4-Phenyl-1,2,3,6-tetrahydropyridyl)-butyl]-5-hydroxymethylindol, F. 178°.

**Beispiel 20**

In eine siedende Lösung von 4,04 g 3-[4-(4-phehyl-1,2,3,6-tetrahydropyridyl)-butyl]-indol-5-carbonsäure in 50 ml absolutem Ethanol wird 2 Std. HCl eingeleitet. Man kocht noch 1 Std., arbeitet wie üblich auf und erhält 3-[4-(4-Phenyl-1,2,3,6-tetrahydropyridyl)-butyl]-indol-5-carbonsäureethylester. Rf 0,65 (Kieselgel; $CH_2Cl_2/CH_3OH$ 8 : 2).

Analog erhält man durch Veresterung:
3-[4-(4-Phenyl-1,2,3,6-tetrahydropyridyl)-butyl]-indol-2-carbonsäuremethylester
3-[4-(4-Phenyl-1,2,3,6-tetrahydropyridyl)-butyl]-indol-4-carbonsäuremethylester
3-[4-(4-Phenyl-1,2,3,6-tetrahydropyridyl)-butyl]-indol-5-carbonsäuremethylester
3-[4-(4-Phenyl-1,2,3,6-tetrahydropyridyl)-butyl]-indol-6-carbonsäuremethylester
3-[4-(4-Phenyl-1,2,3,6-tetrahydropyridyl)-butyl]-indol-7-carbonsäuremethylester
3-[4-(4-Phenyl-1,2,3,6-tetrahydropyridyl)-butyl]-indol-2-carbonsäureethylester
3-[4-(4-Phenyl-1,2,3,6-tetrahydropyridyl)-butyl]-indol-4-carbonsäureethylester
3-[4-(4-Phenyl-1,2,3,6-tetrahydropyridyl)-butyl]-indol-6-carbonsäureethylester
3-[4-(4-Phenyl-1,2,3,6-tetrahydropyridyl)-butyl]-indol-7-carbonsäureethylester
3-[4-(4-Phenyl-1,2,3,6-tetrahydropyridyl)-butyl]-5-methoxyindol-6-carbonsäuremethylester
3-[4-(4-Phenyl-1,2,3,6-tetrahydropyridyl)-butyl]-5-methoxyindol-6-carbonsäureethylester
3-[4-(4-Phenyl-1,2,3,6-tetrahydropyridyl)-butyl]-5-hydroxyindol-6-carbonsäuremethylester
3-[4-(4-Phenyl-1,2,3,6-tetrahydropyridyl)-butyl]-5-hydroxyindol-6-carbonsäureethylester
3-[4-(4-Phenyl-1,2,3,6-tetrahydropyridyl)-butyl]-7-chlorindol-4-carbonsäuremethylester, Hydrochlorid, F.219-221°
3-[4-(4-Phenyl-1,2,3,6-tetrahydropyridyl)-butyl]-7-chlorindol-4-carbonsäureethylester
3-[4-(4-Phenyl-1,2,3,6-tetrahydropyridyl)-butyl]-indol-5-carbonsäurebutylester.

**Beispiel 21**

Man löst 4,04 g 3-[4-(4-Phenyl-1,2,3,6-tetrahydropyridyl)-butyl]-indol-5-carbonsäure in 30 ml Chloroform, sättigt mit HCl-Gas, tropft 1,8 g Thionylchlorid hinzu und kocht 2 Std. Man dampft ein, gibt 30 ml Toluol hinzu und dampft erneut ein. Man löst den Rückstand in 20 ml Chloroform, tropft diese Lösung unter Rühren zu einer gesättigten Lösung von Ammoniak in 50 ml Chloroform, rührt 2 Std. bei 20°, filtriert und konzentriert das Filtrat. Nach üblicher Aufarbeitung erhält man 3-[4-(4-Phenyl-1,2,3,6-tetrahydropyridyl)-butyl]-indol-5-carboxamid, F. 207-208°.

Analog erhält man aus den Säuren durch Umsetzung mit $SOCl_2$, dann Ammoniak bzw. den entsprechenden Aminen:

3-[4-(4-Phenyl-1,2,3,6-tetrahydropyridyl)-butyl]-indol-2-carboxamid
3-[4-(4-Phenyl-1,2,3,6-tetrahydropyridyl)-butyl]-indol-2-carbonsäure-N-methylamid
3-[4-(4-Phenyl-1,2,3,6-tetrahydropyridyl)-butyl]-indol-2-carbonsäure-N,N-dimethylamid. Rf 0,72 (Kieselgel, $CH_2Cl_2/CH_3OH$ 8 : 2).

**Beispiel 22**

Zu einer Lösung von 4,18 g 3-[4-(4-Phenyl-1,2,3,6-tetrahydropyridyl)-butyl]-indol-5-carbonsäuremethylester in 30 ml Dimethylformamid werden 0,02 mol konz. Ammoniak (D = 0,9) bei 20° zugetropft. Man rührt 1 Std. bei 20° nach, arbeitet wie üblich auf und erhält 3-[4-(4-Phenyl-1,2,3,6-tetrahydropyridyl)-butyl]-indol-5-carbonsäureamid. F. 207-208°.

**Beispiel 23**

Ein Gemisch von 37,3 g 3-[4-(4-Phenyl-1,2,3,6-tetrahydropyridyl)-butyl]-5-carbamoylindol, 27,1 g NaOH, 525 ml Wasser und 450 ml Diethylenglykolmonoethylether wird 16 Std. unter Rühren gekocht. Man kühlt ab, arbeitet wie üblich auf, säuert an und erhält 3-[4-(4-Phenyl-1,2,3,6-tetrahydropyridyl)-butyl]-indol-5-carbonsäure, F. 284-285°.

**Beispiel 24**

Man kocht 4,5 g 3-[4-(4-Phenyl-1,2,3,6-tetrahydropyridyl)-2-thiabutyl]-5-methoxy-6-ethoxycarbonylindol 30 Min. mit 20 ml Wasser und 100 ml 2n ethanolischer KOH, arbeitet wie üblich auf und erhält 3-[4-(4-Phenyl-1,2,3,6-tetrahydropyridyl)-2-thiabutyl]-5-methoxyindol-6-carbonsäure, F. 168-171°.

Analog erhält man durch Verseifung der entsprechenden Methyl- oder Ethylester:

3-[4-(4-Phenyl-1,2,3,6-tetrahydropyridyl)-2-thiabutyl]-indol-4-carbonsäure
3-[4-(4-Phenyl-1,2,3,6-tetrahydropyridyl)-2-thiabutyl]-indol-5-carbonsäure, F. 184-189°
3-[4-(4-Phenyl-1,2,3,6-tetrahydropyridyl)-2-thiabutyl]-indol-6-carbonsäure
3-[4-(4-Phenyl-1,2,3,6-tetrahydropyridyl)-2-thiabutyl]-indol-7-carbonsäure
3-[4-(4-Phenyl-1,2,3,6-tetrahydropyridyl)-2-thiabutyl]-5-hydroxyindol-6-carbonsäure
3-[4-(4-Phenyl-1,2,3,6-tetrahydropyridyl)-2-thiabutyl]-7-chlorindol-4-carbonsäure
3-[4-(4-m-Hydroxyphenyl-1,2,3,6-tetrahydropyridyl)-2-thiabutyl]-indol-5-carbonsäure
3-[4-(4-p-Hydroxyphenyl-1,2,3,6-tetrahydropyridyl)-2-thiabutyl]-indol-5-carbonsäure
3-[4-(3-m-Hydroxyphenyl-1,2,3,6-tetrahydropyridyl)-2-thiabutyl]-indol-5-carbonsäure
3-[4-(3-m-Hydroxyphenyl-1,2,3,6-tetrahydropyridyl)-2-thiabutyl]-indol-6-carbonsäure
3-[4-(3-p-Hydroxyphenyl-1,2,3,6-tetrahydropyridyl)-2-thiabutyl]-indol-5-carbonsäure
3-[4-(3-p-Hydroxyphenyl-1,2,3,6-tetrahydropyridyl)-2-thiabutyl]-indol-6-carbonsäure
3-[4-(4-Phenylpiperidino)-2-thiabutyl]-indol-5-carbonsäure
3-[4-(4-Phenylpiperidino)-2-thiabutyl]-indol-6-carbonsäure
3-[4-(4-m-Hydroxyphenylpiperidino)-2-thiabutyl]-indol-5-carbonsäure
3-[4-(4-m-Hydroxyphenylpiperidino)-2-thiabutyl]-indol-6-carbonsäure
3-[4-(4-p-Hydroxyphenylpiperidino)-2-thiabutyl]-indol-5-carbonsäure
3-[4-(4-p-Hydroxyphenylpiperidino)-2-thiabutyl]-indol-6-carbonsäure
3-[4-(3-m-Hydroxyphenylpiperidino)-2-thiabutyl]-indoi-5-carbonsäure
3-[4-(3-m-Hydroxyphenylpiperidino)-2-thiabutyl]-indol-6-carbonsäure
3-[4-(3-m-Hydroxyphenylpiperidino)-2-thiabutyl]-7-chlorindol-4-carbonsäure
3-[4-(3-p-Hydroxyphenylpiperidino)-2-thiabutyl]-7-chlorindol-5-carbonsäure
3-[4-(3-p-Hydroxyphenylpiperidino)-2-thiabutyl]-7-chlorindol-6-carbonsäure.

**Beispiel 25**

Analog Beispiel 1 erhält man aus den entsprechenden Ausgangsstoffen der Formeln II und III:
3-[3-(4-Phenyl-1,2,3,6-tetrahydropyridyl)-propyl]-4-hydroxymethylindol, F. 164-168°
2-Hydroxymethyl-3-[4-(4-phenyl-1,2,3,6-tetrahydropyridyl)-butyl]-5-methoxyindol, F. 145-146°
2-Hydroxymethyl-3-[4-(4-phenyl-1,2,3,6-tetrahydropyridyl)-butyl]-6-methoxyindol.

**Beispiel 26**

Analog Beispiel 3 erhält man aus den entsprechenden Oxocarbonsäuren:
3-[2-(4-Phenyl-1,2,3,6-tetrahydropyridyl)-ethyl]-indol-6-carbonsäure, F. > 240°
3-[3-(4-Phenyl-1,2,3,6-tetrahydropyridyl)-propyl]-indol-4-carbonsäure, F. 268-271°
3-[4-(4-Phenyl-1,2,3,6-tetrahydropyridyl)-butyl]-5-hydroxyindol-6-carbonsäure
3-[4-(4-Phenyl-1,2,3,6-tetrahydropyridyl)-butyl]-6-hydroxyindol-5-carbonsäure
3-[4-(4-Phenyl-1,2,3,6-tetrahydropyridyl)-2-thiabutyl]-5-hydroxyindol-6-carbonsäure
3-[4-(4-Phenyl-1,2,3,6-tetrahydropyridyl)-2-thiabutyl]-6-hydroxyindol-5-carbonsäure.

**Beispiel 27**

Analog Beispiel 8 erhält man aus 1-(2-Mercaptoethyl)-3-m-hydroxyphenyl-piperidin und 5-Hydroxymethylgramin das 3-[4-(3-m-Hydroxyphenyl-piperidyl)-2-thiabutyl]-5-hydroxymethylindol.

**Beispiel 28**

Eine Suspension von 3,9 g 2-Hydroxymethyl-3-[4-(4-phenyl-1,2,3,6-tetrahydropyridyl)-butyl]-6-methoxyindol in 400 ml Toluol wird mit einer Lösung von 1,5 g Diisobutylaluminiumhydrid in 15 ml Toluol unter Kühlung und Rühren versetzt. Man läßt auf Raumtemperatur kommen, kocht 3 Std. unter Rühren, kühlt ab, arbeitet wie üblich auf und erhält 2-Hydroxymethyl-3-[4-(4-phenyl-1,2,3,6-tetrahydropyridyl)-butyl]-6-hydroxyindol.

Analog erhält man aus der entsprechenden 5-Methoxyverbindung das 2-Hydroxymethyl-3-[4-(4-phenyl-1,2,3,6-tetrahydropyridyl)-butyl]-5-hydroxyindol.

Die nachstehenden Beispiele betreffen pharmazeutische Zubereitungen, die Amine der Formel I oder ihre Säureadditionssalze enthalten:

**Beispiel A: Tabletten**

Ein Gemisch von 1 kg 3-[4-(4-Phenyl-1,2,3,6-tetrahydropyridyl)-butyl]-indol-5-carboxamid, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel B: Dragees**

Analog Beispiel A werden Tabletten gepreßt, die anschließend in üblicher Weise mit einem Überzug aus Saccharose, Kartoffelstärke, Talk, Tragant und Farbstoff überzogen werden.

**Beispiel C: Kapseln**

2 kg 3-[4-(4-Phenyl-1,2,3,6-tetrahydropyridyl)-butyl]-indol-5-carbonsäure werden in üblicher Weise in Hartgelatinekapseln gefüllt, so daß jede Kapsel 20 mg des Wirkstoffs enthält.

**Beispiel D: Ampullen**

Eine Lösung von 1 kg 3-[4-(4-Phenyl-1,2,3,6-tetrahydropyridyl)-butyl]-5-methoxyindol-6-carbonsäure-hydrochlorid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Indolderivate der allgemeinen Formel I

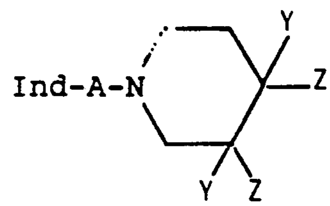

worin

Ind einen durch eine Hydroxymethyl- oder COW-Gruppe substituierten Indol-3-ylrest, der zusätzlich ein- oder zweifach durch Alkyl, O-Alkyl, OH, F, Cl oder Br substituiert sein kann,

W H, OH, OAlkyl, $NH_2$, NHAlkyl oder $N(Alkyl)_2$,

A $-(CH_2)_n-$,$-CH_2-S-CH_2-CH2-$, $-CH_2-SO-CH_2-CH_2$ oder $-CH_2-SO_2-CH_2-CH_2$,

n 2 3, 4 oder 5,

die beiden Reste Y jeweils H oder zusammen eine C-C-Bindung,

der eine Rest Z Ar,

der andere Rest Z H und

Ar eine unsubstituierte oder eine ein- oder zweifach durch O-Alkyl und/oder OH oder durch eine Methylendioxygruppe substituierte Phenylgruppe oder eine 2- oder 3-Thienylgruppe bedeuten,

worin die Alkylgruppen jeweils 1 - 4 C-Atome besitzen,

worin jedoch n nur dann 2 oder 3 bedeutet, wenn gleichzeitig die Hydroxymethyl- oder COW-Gruppe in der 4-, 5-, 6- oder 7-Stellung des Indol-3-ylrests steht,

sowie deren physiologisch unbedenkliche Säureadditionssalze.

2. a) 3-[4-(4-Phenyl-1,2,3,6-tetrahydropyridyl)-butyl]-indol-5-carbonsäure.

b) 3-[4-(4-Phenyl-1,2,3,6-tetrahydropyridyl)-butyl]-indol-5-carboxamid.

3. Verfahren zur Herstellung von Indolderivaten der allgemeinen Formel I nach Anspruch 1 sowie von deren physiologisch unbedenklichen Säureadditionssalzen, dadurch gekennzeichnet, daß man eine Verbindung der allgemeinen Formel II

$Ind-A-X^1$ II

worin

$X^1$ X oder $NH_2$ und

X Cl, Br, J, OH oder eine reaktionsfähig funktionell abgewandelte OH-Gruppe bedeuten und

Ind und A die angegebenen Bedeutungen haben, mit einer Verbindung der allgemeinen Formel III

$X^2-CH_2CH_2-CYZ-CYZ-CH_2-X^3$ III

worin

$X^2$ und $X^3$ gleich oder verschieden sein können und, falls $X^1 = NH_2$ ist, jeweils X, andernfalls zusammen NH bedeuten und

Y und Z die angegebenen Bedeutungen haben, umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt

oder daß man eine sonst der Formel I entsprechende Verbindung, die jedoch an Stelle eines oder mehrerer Wasserstoffatome eine oder mehrere solvolysierbare Gruppe(n) enthält, mit einem solvolysierenden Mittel behandelt

oder daß man zur Herstellung von Thioethern der Formel I, worin A $-CH_2-S-CH_2CH_2-$ bedeutet, eine

Verbindung der allgemeinen Formel IV

Ind-$CH_2N(R)_2$ IV

worin
R Alkyl mit 1-4 C-Atomen, beide Reste R zusammen auch -$(CH_2)_p$ - oder -$CH_2CH_2OCH_2CH_2$- und
P 4 oder 5 bedeuten und
Ind die angegebene Bedeutung hat mit einem Thiol der allgemeinen Formel V

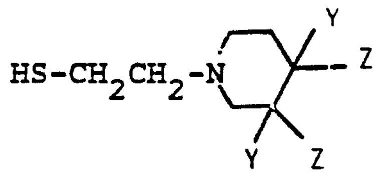

V

worin
Y und Z die angegebene Bedeutungen haben oder einem seiner Salze umsetzt
oder daß man zur Herstellung von Verbindungen der Formel I, worin beide Reste Y zusammen eine C-C-Bindung bedeuten, eine Verbindung der allgemeinen Formel VI

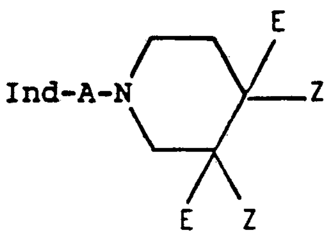

VI

worin
der eine Rest E, X, CN oder $NH_2$
der andere Rest E H bedeutet und
Ind, A, Z und X die angegebenen Bedeutungen haben mit einem HE-abspaltenden Mittel behandelt
und/oder daß man gegebenenfalls in einer Verbindung der Formel I eine Thioerhergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder eine COW-Gruppe durch Oxydation, Reduktion, Veresterung, Amidierung oder Solvolyse in eine andere COW-Gruppe umwandelt und/oder eine COW-Gruppe zu einer Hydroxymethylgruppe reduziert und/oder eine Hydroxymethylgruppe zu einer CHO- oder COOH-Gruppe oxydiert und/oder daß man eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man eine Verbindung der Formel I nach Patentanspruch 1 und/oder eines ihrer physiologisch unbedenklichen Säureadditionssalze zusammen mit mindestens einem festen, flüssigen oder halbflüssigen Träger- oder Hilfsstoff und gegebenenfalle in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I nach Patentanspruch 1 und/oder einem ihrer physiologisch unbedenklichen Säureadditionssalze.

6. Verbindungen der allgemeinen Formel I nach Patentanspruch 1 zur Bekämpfung von Krankheiten.

7. Verwendung von Verbindungen der allgemeinen Formel I nach Patentanspruch 1 zur Herstellung pharmazeutischer Zubereitungen.

## Claims

1. Indole derivatives of the general formula

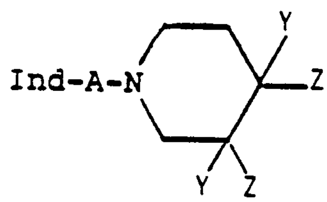

wherein

Ind is an indol-3-yl radical which is substituted by a hydroxymethyl or COW group and which can additionally be monosubstituted or disubstituted by alkyl, O-alkyl, OH, F, Cl or Br,

W is H, OH, Oalkyl, $NH_2$, NHalkyl or $N(alkyl)_2$,

A is $-(CH_2)_n-$, $-CH_2-S-CH_2CH_2-$, $-CH_2-SO-CH_2CH_2-$or $-CH_2-SO_2-CH_2CH_2-$,

n is 2, 3, 4 or 5,

the two radicals Y are each H or together are a C-C bond, one radical Z is Ar, the other radical Z is H and

Ar is a phenyl group which is unsubstituted or is monosubstituted or disubstituted by O-alkyl and/or OH or is substituted by a methylenedioxy group, or Ar is a 2-thienyl or 3-thienyl group,

in which formula each of the alkyl groups has 1 - 4 C atoms, wherein, however, when n is 2 or 3, the hydroxymethyl or COW group must be in the 4-, 5-, 6- or 7-position of the indol-3-yl radical,

and also physiologically acceptable acid addition salts thereof.

2. a) 3-[4-(4-Phenyl-1,2,3,6-tetrahydropyridyl)-butyl]-indole-5-carboxylic acid.

b) 3-[4-(4-Phenyl-1,2,3,6-tetrahydropyridyl)-butyl]-indole-5-carboxamide.

3. Process for the preparation of indole derivatives of the general formula I according to Claim 1 and physiologically acceptable acid addition salts thereof, characterised in that a compound of the general formula

Ind-A-$X^1$ II

wherein

$X^1$ is X or $NH_2$ and

X is Cl, Br, I, OH or a reactive, functionally modified OH group, and

Ind and A have the meanings indicated, is reacted with a compound of the general formula

$X^2-CH_2CH_2-CYZ-CYZ-CH_2-X^3$ III

wherein

$X^2$ and $X^3$ can be identical or different and, if $X^1$ is $NH_2$, are each X and otherwise are together NH and

Y and Z have the meanings indicated,

or a compound which otherwise corresponds to the formula I, but which contains one or more reducible group(s) and/or one or more additional C-C and/or C-N bond(s) instead of one or more hydrogen atoms, is treated with a reducing agent, or a compound which otherwise corresponds to the formula I, but which contains one or more solvolysable group(s) instead of one or more hydrogen atoms, is treated with a solvolytic agent, or, in order to prepare thioethers of the formula I wherein a is $-CH_2-S-CH_2CH_2-$, a compound of the general formula IV

Ind-$CH_2N(R)_2$ IV

wherein

R is alkyl having 1-4 C atoms or both radicals R together are also $-(CH_2)_p-$ or $-CH_2CH_2OCH_2CH_2-$ and

p is 4 or 5 and

Ind has the meaning indicated is reacted with a thiol of the general formula V

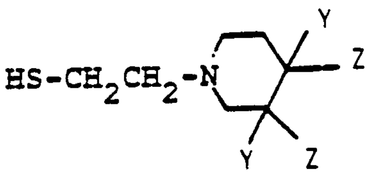

V

wherein
Y and Z have the meanings indicated,
or with one of its salts,
or, in order to prepare compounds of the formula I wherein both radicals Y together are a C-C bond, a compound of the general formula VI

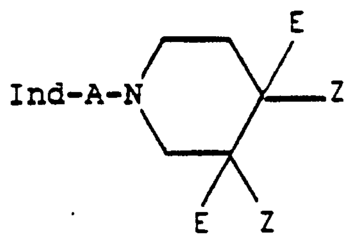

VI

wherein
one radical E is X, CH or $NH_2$,
the other radical E is H and
Ind, A, Z and X have the meanings indicated,
is treated with an agent which splits off HE,
and/or in a compound of the formula I, if appropriate, a thioether group is oxidised to give an SO group or $SO_2$ group or an SO group is oxidised to give an $SO_2$ group and/or an alkoxy group is split with the formation of an OH group and/or a COW group is converted into another COW group by oxidation, reduction, esterification, amidation or solvolysis and/or a COW group is reduced to give a hydroxymethyl group and/or a hydroxymethyl group is oxidised to give a CHO or COOH group and/or a resulting base of the formula I is converted, by treatment with an acid, into one of its physiologically acceptable acid addition salts.

4. Process for the preparation of pharmaceutical formulations, characterised in that a compound of the formula I according to Patent Claim 1 and/or one of its physiologically acceptable acid addition salts is brought into a suitable dosage form together with at least one solid, liquid or semiliquid carrier or auxiliary and, if appropriate, in combination with one or more further active compounds.

5. Pharmaceutical formulation characterised in that it contains at least one compound of the general formula I according to Patent Claim 1 and/or one of its physiologically acceptable acid addition salts.

6. Compounds of the general formula I according to Patent Claim 1 for combaring diseases.

7. The use of compounds of the general formula I according to Patent Claim 1 for the manufacture of pharmaceutical formulations.

**Revendications**

1. Dérivés d'indole de formule générale

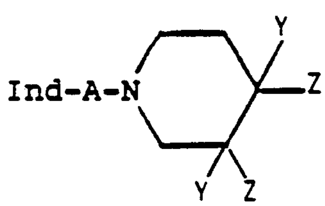

dans laquelle

Ind représente un radical indol-3-yle substitué par un groupe hydroxyméthyle ou COW, qui en outre peut être mono- ou disubstitué par un alcoyle, O-alcoyle, OH, F, Cl ou Br,

W représente H, OH, OAlcoyle, $NH_2$, NHAlcoyle ou $N(alcoyle)_2$,

A représente $-(CH_2)_n-$, $-CH_2-S-CH_2CH_2-$, $-CH_2-SO-CH_2CH_2-$ou $-CH_2-SO_2-CH_2CH_2-$,

n vaut 2, 3, 4 ou 5,

les deux radicaux Y représentent chacun H ou ensemble une liaison C-C,
l'un des radicaux Z représente Ar,
l'autre radical Z représente H et
Ar représente un groupe phényle non substitué ou mono- ou bisubstitué par un O-alcoyle et/ou OH ou par un groupe méthylènedioxy, ou un groupe 2- ou 3-thiényle,
où
les groupes alcoyle possèdent chacun de 1 à 4 atomes de carbone,
où cependant n ne vaut 2 ou 3 que lorsque simultanément le groupe hydroxyméthyle ou COW est en position 4, 5, 6 ou 7 du radical indol-3-yle,
ainsi que leurs sels d'addition d'acide physiologiquement acceptables.

2. a) Acide 3-[4-(4-phényl-1,2,3,6-tétrahydropyridyl)-butyl]-indol-5-carboxylique.
b) 3-[4-(4-phényl-1,2,3,6-tétrahydropyridyl)-butyl],-indol-5-carboxamide.

3. Procédé de préparation de dérivés d'indol de formule générale I selon la revendication 1 ainsi que de leurs sels d'addition d'acide physiologiquement acceptables, caractérisé en ce qu'on fait réagir un composé de formule générale II

Ind-A-$X^1$ (II)

où
$X^1$ représente X ou $NH_2$ et
X représente Cl, Br, I, OH ou un groupe OH fonctionnellement modifié réactif et
Ind et A ont les significations indiquées, avec un composé de formule générale III

$X^2$-$CH_2CH_2$-CYZ-CYZ-$CH_2$-$X^3$ (III)

où
$X^2$ et $X^3$ peuvent être identiques ou différents et, si $X^1 = NH_2$, représentent à chaque fois X, ou encore ensemble NH et
Y et Z ont les significations indiquées,
ou en ce qu'on traite un composé correspondant sinon à la formule I, mais contenant cependant à la place d'un ou plusieurs atomes d'hydrogène un ou plusieurs groupes réductibles et/ou une ou plusieurs liaisons C-C et/ou C-N supplémentaires, avec un agent réducteur
ou en ce qu'on traite un composé correspondant sinon à la formule I, qui contient cependant à la place d'un ou plusieurs atomes d'hydrogène un ou plusieurs groupes solvolysables, avec un agent solvolysant
ou en ce que pour préparer des thioéthers de formule I où A représente -$CH_2$-S-$CH_2CH_2$- on fait réagir un composé de formule générale IV

Ind-$CH_2N(R)_2$ (IV)

où
R représente un alcoyle en $C_1$ à $C_4$, les deux radicaux R représentent ensemble également -$(CH_2)$ - ou -$CH_2CH_2OCH_2CH_2$- et
p vaut 4 ou 5
et
Ind a la signification indiquée
avec un thiol de formule générale V

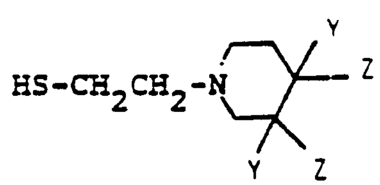

V

où
Y et Z ont les significations indiquées
ou un de ses sels
ou en ce que pour préparer des composés de formule I où les deux radicaux Y représentent ensemble une liaison C-C, on traite un composé de formule générale VI

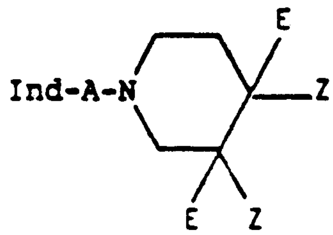

VI

où
l'un des radicaux E représente X, CN ou $NH_2$
l'autre radical E représente H et
Ind, A, Z et X ont les significations indiquées, avec un agent clivant HE
et/ou en ce que si on le désire on oxyde dans un composé de formule I un groupe thioéther en un groupe SO ou un groupe $SO_2$, ou un groupe SO en un groupe $SO_2$ et/ou on clive un groupe alcoxy avec formation d'un groupe OH et/ou on transforme un groupe COW par oxydation, réduction, estérification, amidation ou solvolyse en un autre groupe COW et/ou on réduit un groupe COW en un groupe hydroxyméthyle et/ou on oxyde un groupe hydroxyméthyle en un groupe CHO ou COH et/ou en ce qu'on transforme une base de formule I obtenue par traitement avec un acide en un de ses sels d'addition d'acide physiologiquement acceptables.

4. Procédé de préparation de prépartions pharmaceutiques, caractérisé en ce qu'on met sous une forme posologique appropriée un composé de formule I selon la revendication 1 et/ou un de ses sels d'addition d'acide physiologiquement acceptables avec au moins un support ou additif solide, liquide ou semi-liquide et éventuellement en combinaison avec une ou plusieurs autres substances actives.

5. Préparation pharmaceutique caractérisée en ce qu'elle contient au moins un composé de formule générale I selon la revendication 1 et/ou un de ses sels d'addition d'acide physiologiquement acceptables.

6. Composés de formule générale I selon la revendication 1 en vue de combattre les maladies.

7. Application de composés de formule générale I selon la revendication 1 à la préparation de préparations pharmaceutiques.